(19) Europäisches Patentamt
European Patent Office
Office européen des brevets

(11) Numéro de publication : **0 320 402 B1**

# (12) FASCICULE DE BREVET EUROPEEN

(45) Date de publication du fascicule du brevet : **06.05.92 Bulletin 92/19**

(51) Int. Cl.$^5$ : **C07C 69/757,** C07C 62/16, C07D 317/30

(21) Numéro de dépôt : **88403136.0**

(22) Date de dépôt : **09.12.88**

## (54) Procédé énantiosélectif pour préparer des dérivés de l'aldéhyde hémicaronique de structure trans ou cis et nouveaux produitsintermédiaires obtenus.

(30) Priorité : **11.12.87 FR 8717296**

(43) Date de publication de la demande : **14.06.89 Bulletin 89/24**

(45) Mention de la délivrance du brevet : **06.05.92 Bulletin 92/19**

(84) Etats contractants désignés : **AT BE CH DE FR GB IT LI LU NL**

(56) Documents cités : **DE-A- 3 229 537**

(73) Titulaire : **ROUSSEL-UCLAF**
**35, Boulevard des Invalides**
**F-75007 Paris (FR)**

(72) Inventeur : **Krief, Alain**
**34, Rue Tienne aux Cloches**
**Wepion B 5150 (BE)**
Inventeur : **Dumont, Willy**
**18, Rue des Bruyères**
**B-5190 Salet Anhee (BE)**

(74) Mandataire : **Tonnellier, Marie-José et al**
**111, route de Noisy B.P. no 9**
**F-93230 Romainville (FR)**

**EP 0 320 402 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un nouveau procédé énantiosélectif pour préparer des dérivés de l'aldéhyde hémicaronique de structure trans ou cis, ainsi que des produits intermédiaires nouveaux.

L'invention a ainsi pour objet un procédé de préparation des composés de formule (I) :

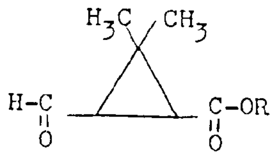

(I)

de structure cis ou trans, racémiques ou optiquement actifs, dans laquelle R représente un atome d'hydrogène, un radical alcoyle renfermant de 1 à 4 atomes de carbone ou un radical aryle renfermant jusqu'à 12 atomes de carbone caractérisé en ce que l'on soumet le composé de formule (II) :

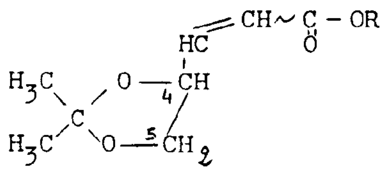

(II)

sous l'une quelconque de ses formes isomères, racémiques ou optiquement actives, dans laquelle R est défini comme précédemment et le trait ondulé schématise une geometrie Z ou E , soit, dans le cas où la geometrie est Z , à l'action d'un agent de gem-diméthyl cyclopropanation pour obtenir le composé de formule (III) :

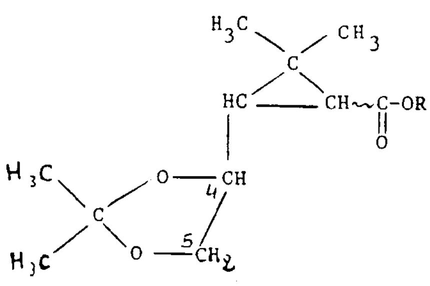

(III)

dans laquelle R et le trait ondulé sont définis comme précédemment, soit dans le cas où la geometrie est E , à l'action d'un agent de gem-diméthyl cyclopropanation différent de l'isopropylidène triphényl phosphorane, pour obtenir le composé de formule (IIIa) :

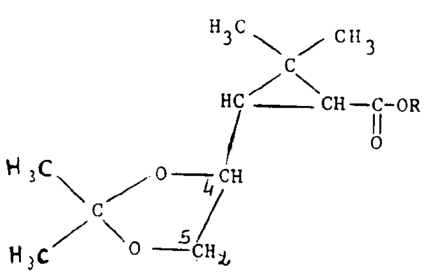

(IIIa)

dans laquelle R est défini comme précédemment et le cycle cyclopropane a la configuration trans, produit de formule (III) ou (IIIa) dont ou bien l'on hydrolyse le reste dioxolanne, pour obtenir le composé de formule (IV) :

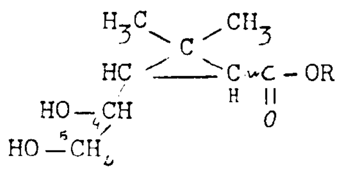

(IV)

dans laquelle R et le trait ondulé sont définis comme précédemment, composé de formule (IV) dont on clive la liaison 4,5 pour obtenir le composé de formule (I) attendu, ou bien l'on hydrolyse le reste dioxolanne et clive simultanément la liaison 4,5 pour obtenir le composé de formule (I) attendu.

R peut représenter un radical méthyle, éthyle, propyle linéaire ou ramifié ou butyle linéaire ou ramifié.

Dans des conditions préférentielles d'exécution du procédé selon l'invention :

– l'agent de gem-diméthyl cyclopropanation du composé de formule (II) de geometrie Z est un réactif de formule :

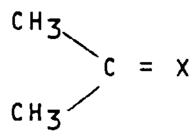

dans laquelle X représente un reste P $(Ar)_3$ , S $(Ar)_2$ , Se (Ar)2 , $As(Ar)_3$ ou

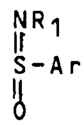

dans laquelle Ar représente un radical aryle et notamment phényle et R représente un atome d'hydrogène ou un radical alcoyle renfermant de 1 à 4 atomes de carbone ;

– l'agent de gem-diméthyl cyclopropanation du composé de formule (II) de geometrie E est un réactif de formule :

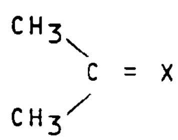

dans laquelle X représente un reste S $(Ar)_2$, Ar étant défini comme précédemment ;

– l'agent d'hydrolyse du reste dioxolanne est un agent acide minéral ou organique, tel que l'acide chlorhydrique, sulfurique, acétique, perchlorique, paratoluène sulfonique ;

– l'agent de clivage de la liaison 4, 5 est un agent oxydant tel qu'un périodate, le tétracétate de plomb ou le permanganate de potassium ;

– l'agent susceptible d'hydrolyser le reste dioxolanne et de cliver la liaison 4,5 est un agent oxydant tel que l'acide périodique ou un périodate en présence d'acide sulfurique.

L'invention a notamment pour objet un procédé de préparation des composés de formule $(I_A)$ :

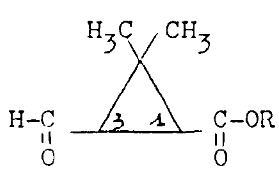

$(I_A)$

de structure trans, racémiques ou optiquement actifs, dans laquelle R est défini comme précédemment, caractérisé en ce que l'on soumet le composé de formule (II) telle que définie précédemment, sous l'une quelconque

de ses formes isomères, racémiques ou optiquement actives, soit dans le cas où la géométrie de la double liaison est Z, à l'action de l'isopropylidène triphényl phosphorane, soit dans le cas où la geometrie de la double liaison est E, à l'action d'un agent de gem-diméthyl cyclopropanation, différent de l'isopropylidène triphényl phosphorane, pour obtenir le composé de formule (IIIa) :

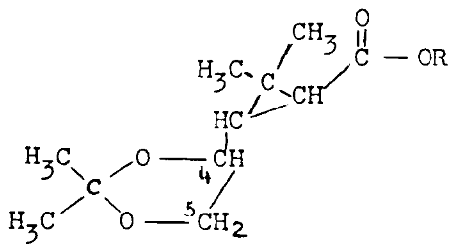

(IIIa)

dans laquelle R est défini comme précédemment et la configuration du cycle cyclopropane est trans, dont soit l'on hydrolyse le reste dioxolanne pour obtenir le composé de formule (IVa) :

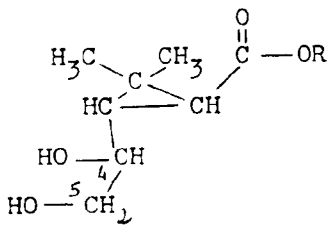

(IVa)

dans laquelle R et la configuration du cycle cyclopropanique sont définis comme précédemment, et clive la liaison 4, 5, pour obtenir le composé de formule ($I_A$) attendu, soit l'on hydrolyse le reste dioxolanne et clive simultanément la liaison 4, 5, pour obtenir le composé de formule ($I_A$) attendu.

L'invention a aussi notamment pour objet un procédé de préparation des composés de formule ($I_B$) :

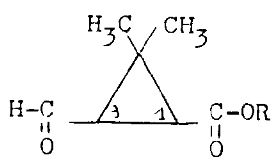

($I_B$)

de structure cis, racémiques ou optiquement actifs, dans laquelle R est défini comme précédemment, caractérisé en ce que l'on soumet le composé de formule (II') :

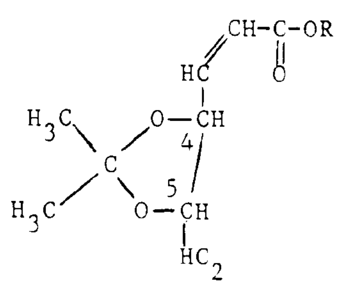

(II')

dans laquelle R est défini comme précédemment et la géométrie de la double liaison est Z, à l'action de l'isopropylidène diphényl sulfurane, pour obtenir le composé de formule (IIIb):

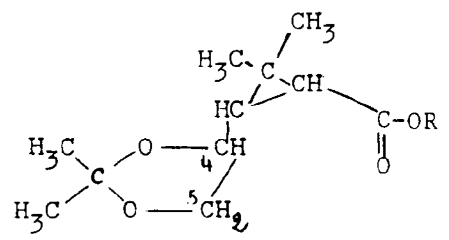

(IIIb)

dans laquelle R est défini comme précédemment et la configuration du cycle cyclopropanique est cis, dont soit l'on hydro lyse le reste dioxolanne pour obtenir le composé de formule (IVb) :

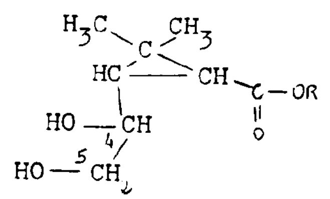

(IVb)

dans laquelle R et la configuration du cycle cyclopropanique sont définis comme précédemment, et clive la liaison 4, 5, pour obtenir le composé de formule ($I_B$) attendu, soit l'on hydrolyse le reste dioxolanne et clive simultanément la liaison 4, 5, pour obtenir le composé de formule (IB) attendu.

L'invention a plus particulièrement pour objet un procédé de préparation des composés de formule (IB) de configuration (1R,cis), caractérisé en ce que l'on soumet le composé de formule ($II'_1$) :

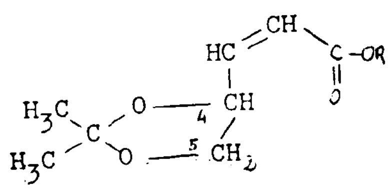

($II'_1$)

de configuration (4S), dans laquelle R est défini comme précédemment et la géométrie de la double liaison est Z, à l'action de l'isopropylidène diphényl sulfurane, pour obtenir le composé de formule ($IIIb_1$) :

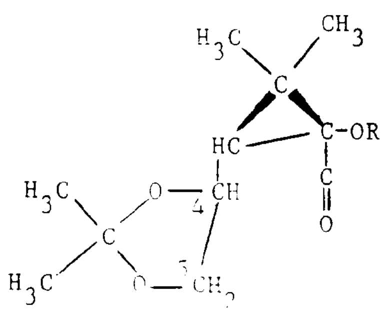

($IIIb_1$)

de configuration (4S) et (1R,cis) au niveau du cycle cyclopropane, dans laquelle R est défini comme précédemment, dont soit l'on hydrolyse le reste dioxolanne, pour obtenir le composé de formule ($IVb_1$) :

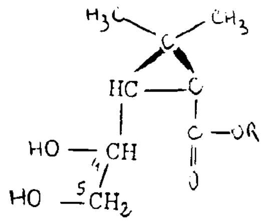

($IVb_1$)

de configuration (4S) et (1R,cis) au niveau du cycle cyclopropane, dans laquelle R est défini comme précédemment et clive la liaison 4, 5, par action d'un agent oxydant, pour obtenir le composé de formule (IB) de configuration (1R,cis), soit l'on hydrolyse le reste dioxolanne et clive simultanément la liaison 4, 5, pour obtenir le composé de formule (IB) de configuration (1R,cis).

L'invention a aussi plus particulièrement pour objet un procédé de préparation des composés de formule (IA) de configuration (1R,trans), caractérisé en ce que l'on soumet le composé de formule ($II''_1$) :

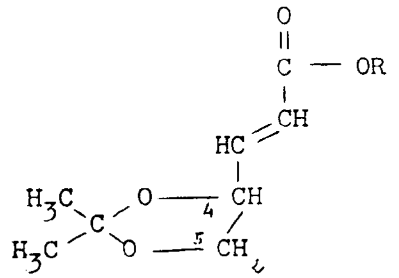

($II''_1$)

de configuration (4R) dans laquelle R est défini comme précédemment et la géométrie de la double liaison est E, que l'on soumet à l'action d'un isopropylidène diphényl sulfurane, pour obtenir le composé de formule ($IIIa_1$) :

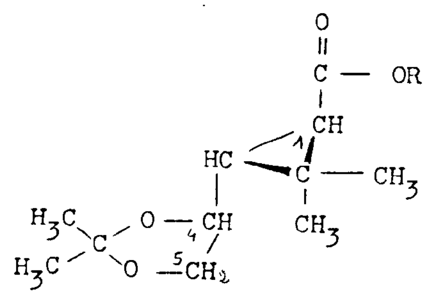

($IIIa_1$)

de configuration (4R) et (1R,trans) au niveau du cycle cyclopropane dans laquelle R est défini comme précédemment, dont soit l'on hydrolyse le reste dioxolanne, pour obtenir le composé de formule ($IVa_1$) :

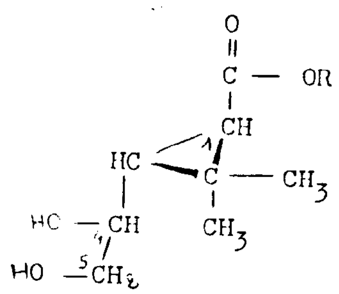

($IVa_1$)

de configuration (4R) et (1R,trans) au niveau du cycle cyclopropane, dans laquelle R est défini comme précédemment et clive la liaison 4,5 par action d'un agent oxydant, pour obtenir le composé de formule (IA) de configuration (1R,trans), soit l'on hydrolyse le reste dioxolanne et clive simultanément la liaison 4,5 pour obtenir le composé de formule (IA) de configuration (1R,trans).

L'invention a aussi plus particulièrement pour objet un procédé de préparation des composés de formule (IA) de configuration 1R,trans, caractérisé en ce que l'on soumet le composé de formule ($II'_1$) :

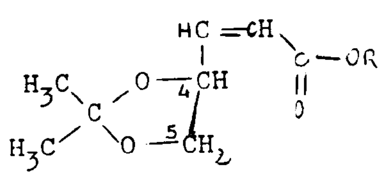

($II'_1$)

de configuration (4R) dans laquelle R est défini comme précédemment et la géométrie de la double liaison est Z, que l'on soumet à l'action d'un isopropylidène triphényl phosphorane pour obtenir le composé de formule ($IIIa_1$) tel que défini ci- dessus, puis poursuit la synthèse comme précédemment pour obtenir le produit de formule (IA) de configuration (1R,trans) attendu.

Outre les composés de formule de structure (1R, cis), selon la configuration en position 4 du composé de départ de formule (II) et selon la configuration de la double liaison des composés de formule (II), le procédé de l'invention permet l'accès énantiosélectif à toutes les structures isomériques possibles de formule (I).

Dans le tableau ci-dessous, ont été représentées les configurations des composés de formule (I) obtenus de géométrie cis ou trans ainsi que des intermédiaires isolés, au départ de composés chiraux de formule (II) en utilisant soit l'isopropylidène triphenylphosphorane , soit l'isopropylidène diphénylsulfurane ; par commodité, la configuration de l'acétonide du glycéraldehyde de départ a aussi été mentionnée.

| Réactif | Acétonide du glycéraldéhyde | Composé (II) | Composé (III) | Composé (I) |
|---|---|---|---|---|
| | D (ou R) | 4S.E | 1R trans 4S | 1R trans |
| $\phi_3P=\!\!<$ | " | 4S.Z | 1S trans 4S | 1S trans |
| | L (ou S) | 4R.E | 1S trans 4R | 1S trans |
| | " | 4R.Z | 1R trans 4R | 1R trans |
| | D (ou R) | 4S.E | 1S trans 4S | 1S trans |
| $\phi_2S=\!\!<$ | " | 4S.Z | 1R cis 4S | 1R cis |
| | L (ou S) | 4R.E | 1R trans 4R | 1R trans |
| | " | 4R.Z | 1S cis 4R | 1S cis |

Il était connu par la demande allemande P 32 29 537.5 de traiter un ester d'alcoyle de l'acide trans-3-(2,2-diméthyl-1,3-dioxolan-4-yl)-acrylique par l'isopropylidène triphényl phosphorane pour obtenir l'ester correspondant d'acide 1R,trans-2,2-diméthyl-3-(2,2-diméthyl-1,3-dioxolan-4-yl) cyclopropane carboxylique.

Cette demande, dirigée vers l'obtention de la seule série trans, n'envisage pas la possibilité d'utiliser d'autres phosphoranes que l'isopropylidène triphényl phosphorane. Elle n'envisage pas non plus la possibilité d'obtenir des esters de la série cis et elle est en ce sens limitée, car, de toute manière, le phosphorane qu'elle utilise ne permet pas d'accéder à ces esters car il entraîne une inversion de configuration de l'ester acrylique de départ, inversion d'ailleurs non prévue dans la demande en question. Enfin, elle n'envisage pas non plus la possibilité d'utiliser cette propriété particulière du phosphorane en question, pour accéder à la série trans en partant d'esters acryliques cis.

La présente demande apporte une réponse nouvelle et inventive à chacun des points ci-dessus, grâce à l'utilisation d'un phosphorane particulier et au parti que l'on peut tirer de l'observation de l'inversion de configuration mentionnée plus haut.

L'invention a également pour objet les composés de formules (IIIb) et (IVb) obtenus lors de la mise en oeuvre du procédé de préparation des composés de formule (I) de structure cis.

Les composés de formule (I) connus sous l'appellation d'aldéhyde hémicaronique sont des intermédiaires utiles dans la synthèse de l'acide chrysanthémique ou de ses analogues notamment halogénés.

Les esters de formule (II) sont des composés connus, pouvant être obtenus à partir de l'acetonide du glycéraldéhyde.

Les exemples suivants illustrent l'invention sans toutefois la limiter.

**Exemple 1 : (1R cis) 3-formyl 2,2-diméthyl cyclopropane carboxylate de méthyle.**

**Stade A** : /(4S) 2,2-diméthyl 1,3-dioxolanne 4-yL/ 3-/2,2-diméthyl 1R,3S cyclopropane carboxylate de méthyle/.

A une solution, refroidie à -78¤C et placée sous gaz inerte, de 0,574 g de tétrafluoroborate d'isopropyldiphénylsulfonium et de 0,128 g de dichlorométhane anhydre dans 12 ml de diméthoxyéthane anhydre, on ajoute une solution de diisopropylamidure de lithium, obtenue par traitement durant 15 mn à -78¤C de 0,172 g de diisopropylamine dans 5 ml de diméthoxyéthane par 1,05 ml de n-butyllithium (1,55 M dans l'hexane). On laisse agiter durant 15 minutes à -78¤C, puis on additionne 0,186 g de /(4S) 2,2-diméthyl 1,3-dioxolanne 4-yl/ 3 (Z)-propénoate de méthyle en solution dans 2 ml de diméthoxyéthane. Le mélange réactionnel est agité durant 15 mn à -78¤C puis 45 mn entre -65¤ et -50¤C. On laisse finalement réchauffer durant 15 mn puis on ajoute 5 ml d'eau. La phase organique est diluée dans l'éther, séparée de la phase aqueuse, lavée à l'eau et séchée. L'évaporation des solvants sous vide fournit 0,4 g de produit brut que l'on chromatographie sur silice en éluant au mélange pentane-éther (75-25). On obtient 0,192 g de produit attendu. Alpha $^{20}_{D}$ = -15,4° (c = 18,75% $CHCl_3$).
Spectre RMN ($CCl_4$) Delta 0,9-1,8 (m, 14H, $C\underline{H}_3$ et $\underline{H}$ sur le cyclopropane) ; 3,2-3,65 (m avec s à 3,6 $CO_2C\underline{H}_3$), 4$\underline{H}$, $CO_2$ $C\underline{H}_3$ et un des $\underline{H}$ du dioxolane) ; 3,95 (dd, 1H, un des $\underline{H}$ du dioxolane) ; 4,25-4,66 (m, 1H, un des $\underline{H}$ du dioxolane).

**Stade B** : (1R cis) 3-formyl 2,2-diméthyl cyclopropane carboxylate de méthyle.

On dissout 0,15 g de produit obtenu au Stade A dans 8 ml de THF et on ajoute à 20¤C 4 ml d'une solution aqueuse d'acide perchlorique (2,5 M). On agite durant 3 h à 20¤C. On additionne ensuite du bicarbonate de sodium solide jusqu'à obtention d'un pH basique. On extrait ensuite le mélange réactionnel par l'éther. La phase organique est séchée et les solvants sont évaporés sous vide. On obtient le diol brut qui sera utilisé tel quel pour l'étape suivante.

Le diol brut est dissous dans 6 ml de méthanol et 3 ml de tampon phosphate (pH : 7,2) et traité par un excès de périodate de sodium (soit 0,214 g) additionné en une fois à 20¤C. On agite durant 30 mn à 20¤C, puis on évapore sous vide l'essentiel du méthanol. Le résidu est ensuite agité en présence de 60 ml d'éther. La phase organique est décantée, lavée par 5 ml d'eau et séchée. Après évaporation des solvants sous vide, le mélange brut est purifié par chromatographie sur gel de silice pour fournir 0,06 g de produit attendu. Alpha $_D{}^{20}$ = -76,4¤ (c = 16,9 mg/ml acétone).

**Exemple 2 : (1R trans) 3-formyl 2,2-diméthyl cyclopropane carboxylate de méthyle.**

**Stade A** : /(4R) 2,2-diméthyl 1,3-dioxolanne 4-yl/3-/2,2-diméthyl 1R,3R cyclopropane carboxylate de méthyle/.

On opère comme indiqué au Stade A de l'exemple 1, en utilisant au départ 0,186 g de / (4R) 2,2-diméthyl 1,3-dioxolanne 4-yl/ 3 (E) propénoate de méthyle.

Après chromatographie sur silice en éluant au mélange pentane-éther (7-3), on obtient 0,21 g de produit attendu.

RMN ($CCl_4$) Delta 1,70-0,9 (m avec 2s à 1,2 et 1,35, 12 H, $C\underline{H}_3$ et $C\underline{H}$ cyclopropanique) ; 3,8-4,25 (m avec s à 3,8, 6H, $CO_2$ $CH_3$, $C\underline{H}$-O et $C\underline{H}_2O$).

**Stade B** : (1R trans) 3-formyl 2,2-diméthyl cyclopropane carboxylate de méthyle.

On opère comme indiqué au Stade B de l'exemple 1, en utilisant au départ 0,15 g de produit obtenu au stade A et obtient 0,065 g de produit attendu.

Alpha $_D{}^{20}$ = +13,6¤ (c = 11,5 mg/ml acétone).

**Exemple 3** : (1R trans) 3-formyl 2,2-diméthyl cyclopropane carboxylate de méthyle.

**Stade A** : /(4R) 2,2-diméthyl 1,3-dioxolanne 4-yl/3-/2,2-diméthyl 1R,3R cyclopropane carboxylate de méthyle/.

On prépare une solution (1,5 $10^{-3}$M) de triphénylisopropylidène phosphorane dans le tétrahydrofuranne

par addition d'une solution hexanique de n-butyllithium à une suspension d'iodure d'isopropyltriphénylphosphonium dans le tétrahydrofuranne.

On introduit à 0¤C 6 $cm^3$ de cette solution dans 0,186 g de /(4R) 2,2-diméthyl 1,3-dioxolanne 4-yl/3 (Z) propénoate de méthyle dissous dans 4 $cm^3$ de tétrahydrofuranne et maintient sur agitation 1 heure à 0¤C puis 1 heure après retour à 20¤C. On dilue le milieu réactionnel avec 5 $cm^3$ d'eau, extrait à l'éther, sèche et élimine les solvants sous pression réduite et obtient 0,25 g de produit brut. Après purification par chromatographie sur silice (éluant pentane-éther 8/2), on obtient 0,15 g de produit attendu.

Spectre de RMN ($CCl_4$) :

H de $CO_2$ $CH_3$, -CH-O et -$CH_2$-O : 4,25 à 3,8 ppm(m) ;
H des méthyles et H cyclopropaniques : 1,7 à 0,9 ppm(m).

**Stade B** : (1R trans) 3-formyl 2,2-diméthyl cyclopropane carboxylate de méthyle.

On opère comme indiqué au Stade B de l'exemple 1, en utilisant au départ 0,15 g de produit obtenu ci-dessus et obtient 0,085 g de produit brut que l'on chromatographie sur silice en éluant au mélange pentane-éther (7-3).

Alpha $_D^{20}$ = +13°,6 (c = 11,5 mg/ml acétone).

Exemple 4 : (1S trans) 3-formyl 2,2-diméthyl cyclopropane carboxylate de méthyle.

Stade A : /(4S) 2,2-diméthyl 1,3-dioxolanne 4-yl/3-/2,2-diméthyl 1S,3S cyclopropane carboxylate de méthyle/.

On opère de la même manière qu'à l'exemple 3 en utilisant au départ 0,186 g de /(4S) 2,2-diméthyl 1,3-dioxolanne 4-yl/3 (Z) propénoate de méthyle. On obtient après purification 0,15 g de produit attendu

Alpha $_D^{20}$ = +37,5¤ (c = 13 mg/ml acétone).

**Stade B** : (1S trans) 3-formyl 2,2-diméthyl cyclopropane carboxylate de méthyle.

On opère comme indiqué au Stade B de l'exemple 1 en utilisant au départ 0,15 g de produit obtenu ci-dessus et obtient 0,08 g de produit brut que l'on purifie par chromatographie sur silice en éluant au mélange pentane-éther (7-3).

Alpha $_D^{20}$ = -18,8¤ (c = 13 mg/ml acétone).

Les produits de formule II utilisés au départ des exemples ci-dessus sont décrits, par exemple, par MINAMI.M KOSS KISHI.Y J.A.C.S 1982 (104) p 1109 et MULZER.J KAPPERT.M Angew. Int. Ed. 1983 (22) p 63.

## Revendications

1. Procédé de préparation des composés de formule (I) :

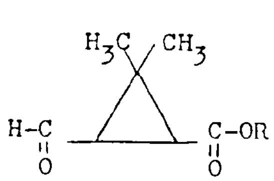

(I)

de structure cis ou trans, racémiques ou optiquement actifs, dans laquelle R représente un atome d'hydrogène, un radical alcoyle renfermant de 1 à 4 atomes de carbone ou un radical aryle renfermant jusqu'à 12 atomes de carbone caractérisé en ce que l'on soumet le composé de formule (II) :

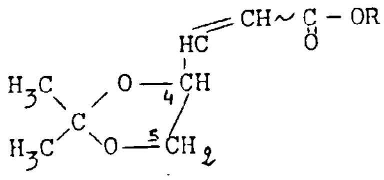

(II)

sous l'une quelconque de ses formes isomères, racémiques ou optiquement actives dans laquelle R est défini comme précédemment et le trait ondulé schématise une géométrie Z ou E, soit dans le cas où la géométrie est Z, à l'action d'un agent de gem-diméthyl cyclopropanation pour obtenir le composé de formule (III) :

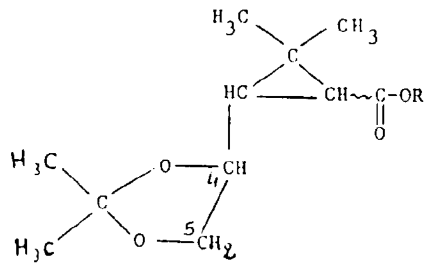

(III)

dans laquelle R et le trait ondulé sont définis comme précédemment soit dans le cas où la géométrie est E, à l'action d'un agent de gem-diméthyl cyclopropanation différent de l'isopropylidène triphényl phosphorane, pour obtenir le composé de formule (IIIa) :

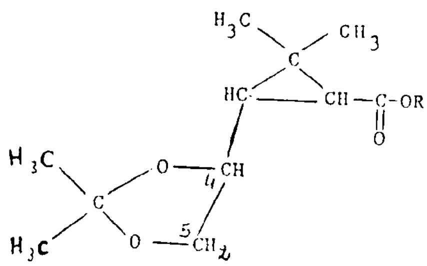

(IIIa)

dans laquelle R est défini comme précédemment et le cycle cyclopropane a la configuration trans, produit de formule (III) ou (IIIa) dont ou bien l'on hydrolyse le reste dioxolanne, pour obtenir le composé de formule (IV) :

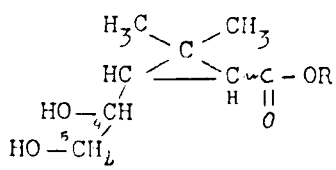

(IV)

dans laquelle R et le trait ondulé sont définis comme précédemment le composé de formule (IV) dont on clive la liaison 4,5 pour obtenir le composé de formule (I) attendu, ou bien l'on hydrolyse le reste dioxolanne et clive simultanément la liaison 4,5 pour obtenir le composé de formule (I) attendu.

2. Procédé selon la revendication 1, caractérisé en ce que :

– l'agent de gem-diméthyl cyclopropanation du composé de formule (II) de géométrie Z est un réactif de formule :

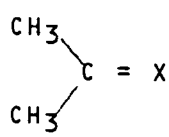

dans laquelle X représente un reste P $(Ar)_3$, $S(Ar)_2$,

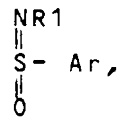

$Se(Ar)_2$ ou $As(Ar)_3$ dans laquelle Ar représente un radical aryle et notamment phényle et $R_1$ représente un atome d'hydrogène ou un radical alcoyle renfermant de 1 à 4 atomes de carbone ;

– l'agent de gem-diméthyl cyclopropanation du composé de formule (II) de géométrie E est un réactif de formule :

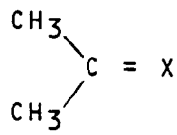

dans laquelle X représente un reste $S(Ar)_2$, Ar étant défini comme précédemment.

3. Procédé selon la revendication 1, pour la préparation des composés de formule (IA) :

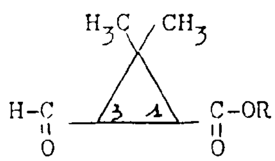

($I_A$)

de structure trans, racémiques ou optiquement actifs, dans laquelle R est défini comme précédemment caractérisé en ce que l'on soumet le composé de formule (II) telle que définie précédemment, sous l'une quelconque de ses formes isomères, racémiques ou optiquement actives, soit, dans le cas où la géométrie de la double liaison est Z, à l'action de l'isopropylidène triphényl phosphorane, soit dans le cas où la géométrie de la double liaison est E à l'action d'un agent de gem-diméthyl cyclopropanation différent de l'isopropylidène triphényl phosphorane, pour obtenir le composé de formule (IIIa) :

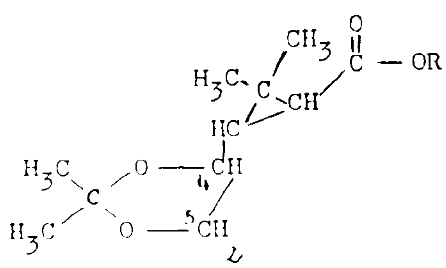

(IIIa)

dans laquelle R est défini comme précédemment et la configuration du cycle cyclopropane est trans, dont soit l'on hydrolyse le reste dioxolanne, pour obtenir le composé de formule (IVa) :

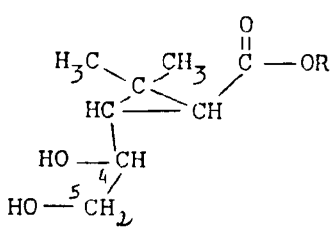

(IVa)

dans laquelle R et la configuration du cycle cyclopropanique sont définis comme précédemment et clive la liaison 4, 5 pour obtenir le composé de formule (IA) attendu soit l'on hydrolyse le reste dioxolanne et clive simultanément la liaison 4,5 pour obtenir le composé de formule (IA) attendu.

4. Procédé selon la revendication 1, pour la préparation des composés de formule (IB) :

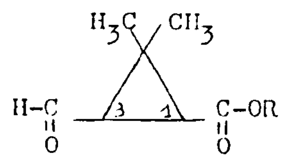

$(I_B)$

de structure cis, racémiques ou optiquement actifs, dans laquelle R est défini comme précédemment, caractérisé en ce que l'on soumet le composé de formule (II') :

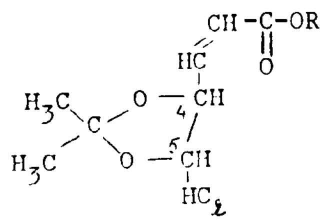

(II')

dans laquelle R est défini comme précédemment et la géométrie de la double liaison est Z, à l'action de l'isopropylidène diphényl sulfurane, pour obtenir le composé de formule (IIIb):

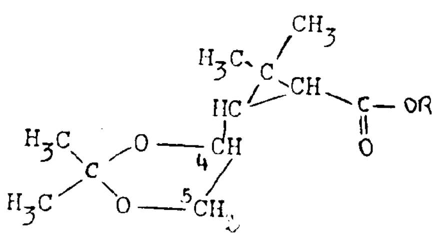

(IIIb)

dans laquelle R est défini comme précédemment et la configuration du cycle cyclopropanique est cis, dont soit l'on hydrolyse le reste dioxolanne pour obtenir le composé de formule (IVb) :

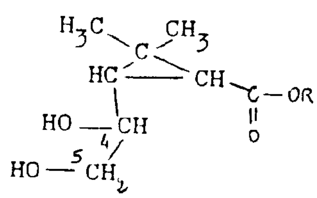

(IVb)

dans laquelle R et la configuration du cycle cyclopropanique sont définis comme précédemment et clive la liaison 4,5 pour obtenir le composé de formule (IB) attendu soit l'on hydrolyse le reste dioxolanne et clive simultanément la liaison 4,5 pour obtenir le composé de formule (IB) attendu.

5. Procédé selon la revendication 4, pour la préparation des composés de formule (IB) de configuration (1R,cis), caractérisé en ce que l'on soumet le composé de formule ($II'_1$) :

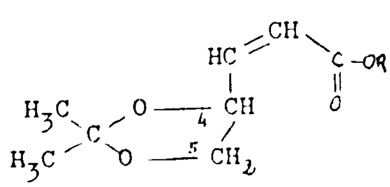

$(II'_1)$

de configuration (4S) dans laquelle R est défini comme précédemment et la géométrie de la double liaison est Z, à l'action de l'isopropylidène diphényl sulfurane, pour obtenir le composé de formule ($IIIb_1$) :

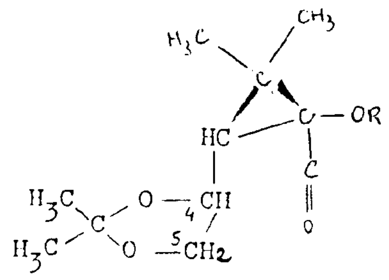

($IIIb_1$)

de configuration (4S) et (1R,cis) au niveau du cycle cyclopropane, dans laquelle R est défini comme précédemment, dont soit l'on hydrolyse le reste dioxolanne, pour obtenir le composé de formule ($IVb_1$) :

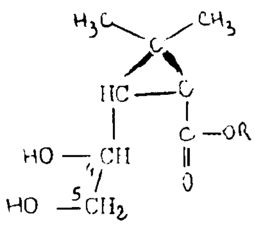

($IVb_1$)

de configuration (4S) et (1R,cis) au niveau du cycle cyclopropane dans laquelle R est défini comme précédemment et clive la liaison 4,5 par action d'un agent oxydant, pour obtenir le composé de formule (IB) de configuration (1R,cis) soit l'on hydrolyse le reste dioxolanne et clive simultanément la liaison 4,5 pour obtenir le composé de formule (IB) de configuration (1R,cis).

6. Procédé selon la revendication 3, pour la préparation des composés de formule (IA) de configuration (1R,trans), caractérisé en ce que l'on soumet le composé de formule ($II''_1$):

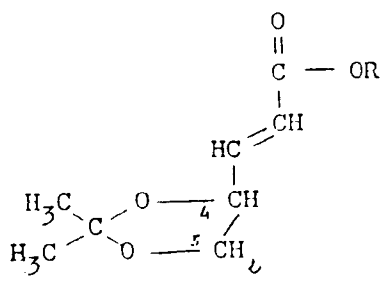

($II''_1$)

de configuration (4R) dans laquelle R est défini comme précédemment et la géométrie de la double liaison est E, que l'on soumet à l'action de l'isopropylidène diphényl sulfurane pour obtenir le composé de formule ($IIIa_1$) :

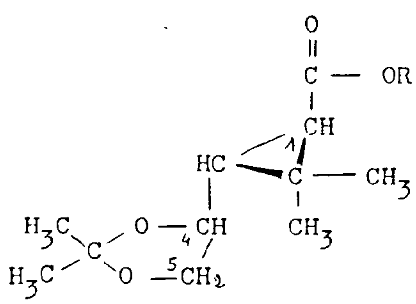

($IIIa_1$)

de configuration (4R) et (1R, trans) au niveau du cycle cyclopropane dans laquelle R est défini comme précédemment dont soit l'on hydrolyse le reste dioxolanne, pour obtenir le composé de formule ($IVa_1$) :

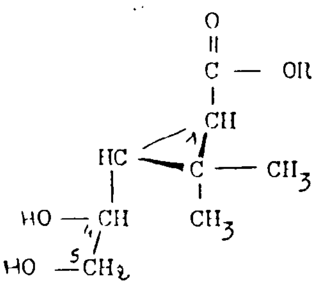

(IVa$_1$)

de configuration (4R) et (1R trans) au niveau des cycles cyclopropanes, dans laquelle R est défini comme précédemment et clive la liaison 4,5 par action d'un agent oxydant, pour obtenir le composé de formule (IA) de configuration (1R,trans), soit l'on hydrolyse le reste dioxolanne et clive simultanément la liaison 4,5 pour obtenir le composé de formule (IA) de configuration (1R trans).

7. Procédé selon la revendication 3, pour la préparation des composés de formule (IA) de configuration (1R,trans) caractérisé en ce que l'on soumet le composé de formule (II'$_1$) :

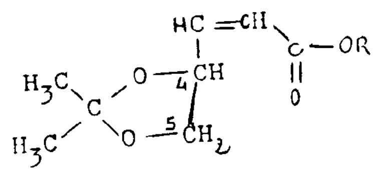

(II'$_1$)

de configuration (4R) dans laquelle R est défini comme précédemment et la géométrie de la double liaison est Z, que l'on soumet à l'action d'un isopropylidène triphényl phosphorane pour obtenir le composé de formule (IIIa$_1$) tel que définie ci-dessus, puis poursuit la synthèse comme à la revendication 6, pour obtenir le produit de formule (IA) de configuration (1R,trans) attendu.

8. A titre de composés industriels nouveaux, les composés de formules (IIIb) et (IVb) telles que définies à la revendication 4.

**Claims**

1. Preparation process for the compounds of formula (I):

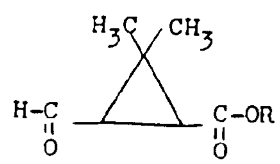

(I)

with cis or trans structure, racemic or optically active, in which R represents a hydrogen atom, an alkyl radical containing 1 to 4 carbon atoms or an aryl radical containing up to 12 carbon atoms characterized in that the compound of formula (II):

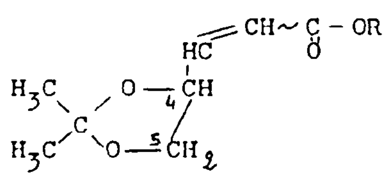

(II)

in any of its racemic or optically active isomer forms in which R is defined as previously and the wavy line represents Z or E geometry, is subjected either in the case where the geometry is Z to the action of a gem-methyl cyclopropanation agent in order to obtain the compound of formula (III):

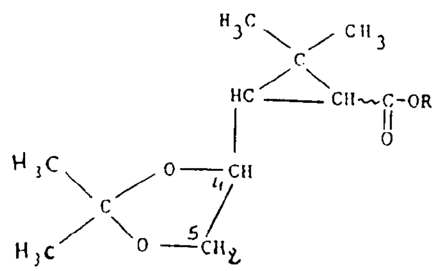

(III)

in which R and the wavy line are defined as previously or in the case where the geometry is E, to the action of a gem-dimethyl cyclopropanation agent different from isopropylidene triphenyl phosphorane, in order to obtain the compound of formula (IIIa):

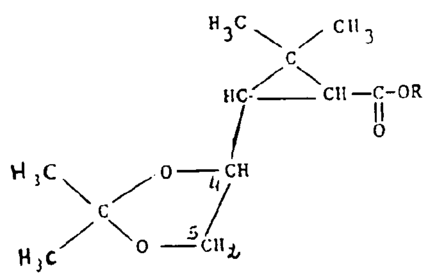

(IIIa)

in which R is defined as previously and the cyclopropane ring is of trans configuration, the dioxolane remainder of which product of formula (III) or (IIIa) either is hydrolysed, in order to obtain the compound of formula (IV):

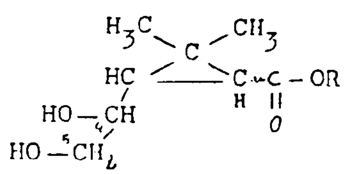

(IV)

in which R and the wavy line are defined as previously, the 4,5 bond of which product of formula (IV) is cleaved in order to obtain the expected compound of formula (I), or the dioxolane remainder is hydrolysed and the 4,5 bond is cleaved simultaneously in order to obtain the expected compound of formula (I).

2. Process according to claim 1, characterized in that:

– the gem-dimethyl cyclopropanation agent of the compound of formula (II) of Z geometry is a reagent of formula:

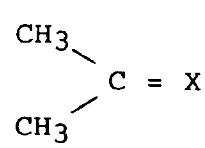

in which X represents a $P(Ar)_3$, $S(Ar)_2$,

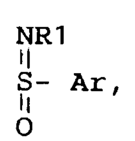

$Se(Ar)_2$ or $As(Ar)_3$ remainder in which Ar represents an aryl radical and notably phenyl and $R_1$ represents a hydrogen atom or an alkyl radical containing 1 to 4 carbon atoms;

– the gem-dimethyl cyclopropanation agent of the compound of formula (II) of E geometry is a reagent of

formula:

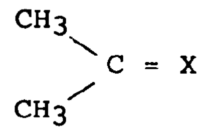

in which X represents an $S(Ar)_2$ remainder, Ar being defined as previously.

3. Process according to claim 1, for the preparation of compounds of formula ($I_A$):

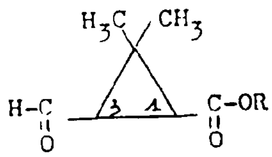

($I_A$)

of trans structure, racemic or optically active, in which R is defined as previously, characterized in that the compound of formula (II) as defined previously, in any of its racemic or optically active isomer forms is subjected, either, in the case where the geometry of the double bond is Z, to the action of isopropylidene triphenyl phosphorane, or, in the case where the geometry of the double bond is E to the action of a gem-dimethyl cyclopropanation agent different from isopropylidene triphenyl phosphorane, in order to obtain the compound of formula (IIIa):

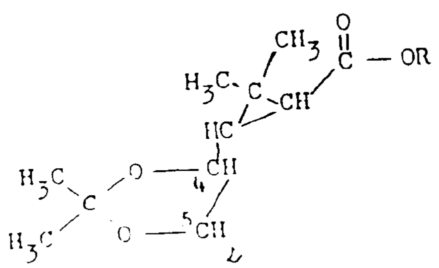

(IIIa)

in which R is defined as previously and the configuration of the cyclopropane ring is trans, of which either the dioxolane remainder is hydrolysed in order to obtain the compound of formula (IVa):

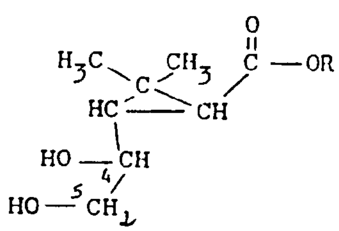

(IVa)

in which R and the configuration of the cyclopropane ring are defined as previously and the 4,5 bond is cleaved in order to obtain the expected compound of formula ($I_A$) or the dioxolane remainder is hydrolysed and the 4,5 bond is cleaved simultaneously in order to obtain the expected compound of formula ($I_A$).

4. Process according to claim 1, for the preparation of the compounds of formula ($I_B$):

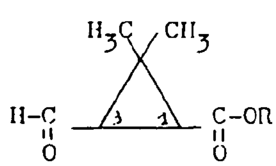

($I_B$)

of cis structure, racemic or optically active, in which R is defined as previously, characterized in that the compound of formula (II'):

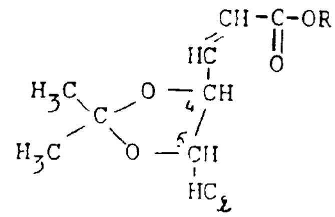

(II')

in which R is defined as previously and the geometry of the double bond is Z, is subjected to the action of isopropylidene diphenyl sulphurane, in order to obtain the compound of formula (IIIb):

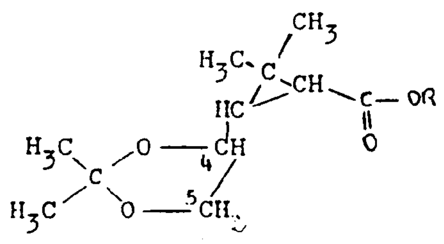

(IIIb)

in which R is defined as previously and the configuration of the cyclopropane ring is cis, of which either the dioxolane remainder is hydrolysed in order to obtain the compound of formula (IVb):

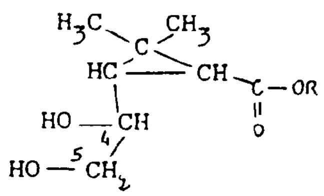

(IVb)

in which R and the configuration of the cyclopropane ring are defined as previously and the 4,5 bond is cleaved in order to obtain the expected compound of formula ($I_B$) or the dioxolane remainder is hydrolysed and the 4,5 bond is cleaved simultaneously in order to obtain the expected compound of formula ($I_B$).

5. Process according to claim 4, for the preparation of compounds of formula ($I_B$) of (1R,cis) configuration, characterized in that the compound of formula ($II'_1$):

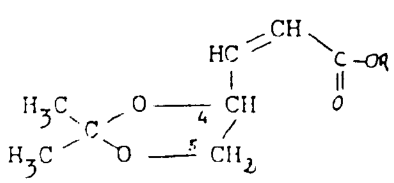

($II'_1$)

of (4S) configuration in which R is defined as previously and the geometry of the double bond is Z, is subjected to the action of isopropylidene diphenyl sulphurane, in order to obtain the compound of formula ($IIb_1$):

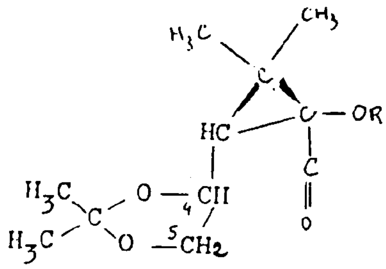

($IIIb_1$)

of (4S) configuration and (1R,cis) configuration at the level of the cyclopropane ring, in which R is defined as previously, of which either the dioxolane remainder is hydrolysed in order to obtain the compound of formula ($ICb_1$):

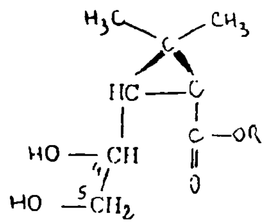

($IVb_1$)

of (4S), configuration and (1R,cis) configuration at the level of the cyclopropane ring in which R is as defined previously and the 4,5 bond is cleaved by the action of an oxidizing agent, in order to obtain the compound of formula ($I_B$) of (1R,cis) configuration or the dioxolane remainder is hydrolysed and the 4,5 bond is cleaved simultaneously in order to obtain the compound of formula ($I_B$) of (1R,cis) configuration.

6. Process according to claim 3, for the preparation of compounds of formula ($I_A$) of (1R,cis) configuration, characterized in that the compound of formula ($II''_1$):

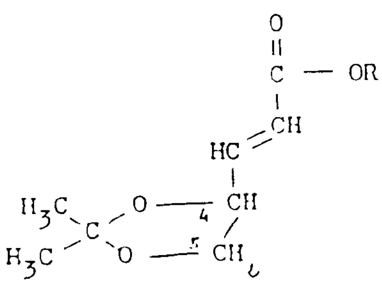

($II''_1$)

of (4R) configuration in which R is defined as previously and the geometry of the double bond is E, is subjected to the action of isopropylidene diphenyl sulphurane in order to obtain the compound of formula ($IIIa_1$):

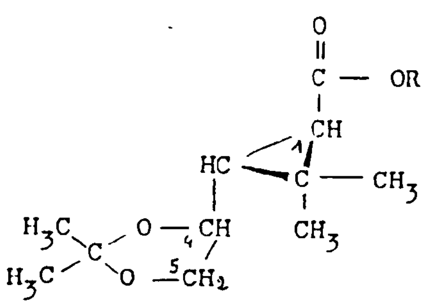

($IIIa_1$)

of (4R) configuration and (1R,trans) configuration at the level of the cyclopropane ring in which R is defined as previously of which either the dioxolane remainder is hydrolysed in order to obtain the compound of formula ($IVa_1$):

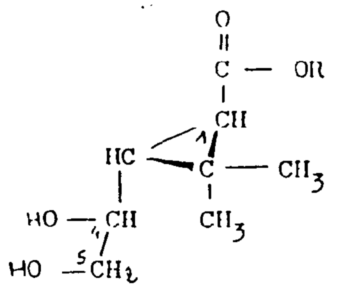

(IVa1)

of (4R) configuration and (1R,cis) configuration at the level of the cyclopropane rings, in which R is defined as previously and the 4,5 bond is cleaved by the action of an oxidizing agent, in order to obtain the compound of formula ($I_A$) of (1R,trans) configuration.

7. Process according to claim 3, for the preparation of compounds of formula ($I_A$) of (1R,trans) configuration characterized in that the compound of formula ($II'_1$):

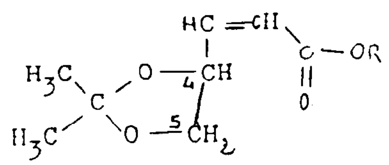

(II'1)

of (4R) configuration in which R is defined as previously and the geometry of the double bond is Z, is subjected to the action of isopropylidene triphenyl phosphorane in order to obtain the compound of formula ($IIIa_1$) as defined above, then the synthesis is continued as in claim 6 in order to obtain the expected product of formula ($I_A$) of (1R,trans) configuration.

8. As new industrial compounds, the compounds of formulae (IIIb) and (IVb) as defined in claim 4.

**Patentansprüche**

1. Verfahren zur Herstellung von racemischen oder optisch aktiven Verbindungen der Formel (I)

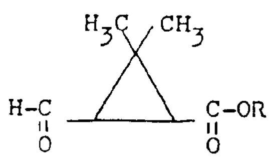

(I)

mit cis- oder trans-Stuktur, worin R ein Wasserstoffatom, einen Alkylrest mit 1 bis 4 Kohlenstoffatomen oder einen Arylrest mit bis zu 12 Kohlenstoffatomen bedeutet, dadurch **gekennzeichnet**, daß man die Verbindung der Formel (II)

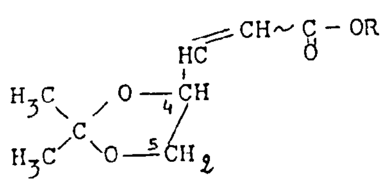

(II)

in irgendeiner ihrer isomeren, racemischen oder optisch aktiven Formen, worin R wie vorstehend definiert ist und die gewellte Linie eine Z- oder E-Geometrie schematisiert, entweder, wenn die Geometrie die Z-Geometrie ist, der Einwirkung eines gem-Dimethylcyclopropanierungssmittels unterzieht, um zu der Verbindung der Formel (III)

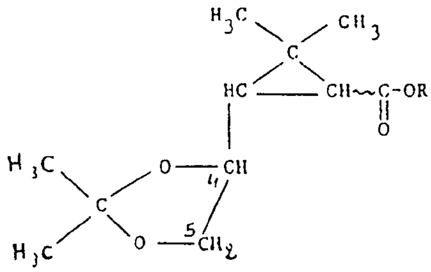

(III)

zu gelangen, worin R und die gewellte Linie die angegebene Bedeutung besitzen, oder, wenn die Geometrie die E-Geometrie ist, der Einwirkung eines gem-Dimethylcyclopropanierungsmittels, das von Isopropyliden-triphenylphosphoran verschieden ist, unterzieht, um die Verbindung der Formel (IIIa)

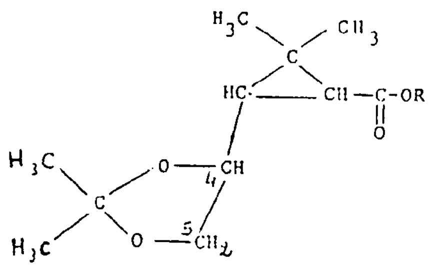

(IIIa)

zu erhalten, worin R wie vorstehend definiert ist und der Cyclopropanring die trans-Konfiguration besitzt, von dem Produkt der Formel (III) oder (IIIa) entweder den Dioxolanrest hydrolysiert, um zu der Verbindung der Formel (IV)

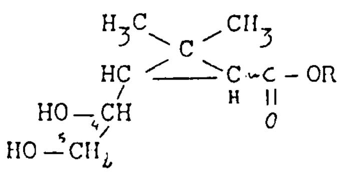

(IV)

zu gelangen, worin R und die gewellte Linie wie vorstehend definiert sind, von der Verbindung (IV) die 4,5-Bindung spaltet, um zu der erwarteten Verbindung der Formel (I) zu gelangen, oder den Dioxolanrest hydrolysiert und gleichzeitig die 4,5-Bindung spaltet, um die erwartete Verbindung der Formel (I) zu erhalten.

2. Verfahren gemäß Anspruch 1, dadurch gekennzeichnet, daß

– das gem-Dimethylcyclopropanierungsmittel der Verbindung der Formel (II) mit Z-Geometrie ein Reagens der Formel

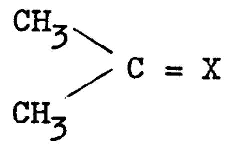

ist, worin X für einen Rest P $(Ar)_3$, $S(Ar)_2$,

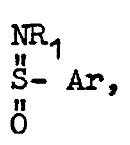

$Se(Ar)_2$ oder $As(Ar)_3$ steht, worin Ar einen Arylrest und insbesondere den Phenylrest bedeutet und $R_1$ ein Wasserstoffatom oder einen Alkylrest mit 1 bis 4 Kohlenstoffatomen wiedergibt,

– das gem-Dimethylcyclopropanierungsmittel der Verbindungen der Formel (II) mit E-Geometrie ein

Reagens der Formel

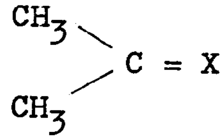

ist, worin X für einen Rest S(Ar)2 steht, wobei Ar wie vorstehend definiert ist.

3. Verfahren gemäß Anspruch 1 zur Herstellung von racemischen oder optisch aktiven Veribindungen der Formel (IA)

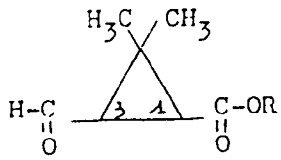

($I_A$)

mit trans-Struktur, worin R wie vorstehend definiert ist, dadurch gekennzeichnet, daß man die Verbindung der Formel (II), wie vorstehend definiert, in einer ihrer isomeren, racemischen oder optisch aktiven Formen entweder, wenn die Geometrie der Doppelbindung die Z-Geometrie ist, der Einwirkung von Isopropyliden-triphenylphosphoran oder, wenn die Geometrie der Doppelbindung die E-Geometrie ist, der Einwirkung eines gem-Dimethylcyclopropanierungsmittels, das von Isopropyliden-triphenylphosphoran oderschieden ist, unterzieht, um zu der Verbindung der Formel (IIIa)

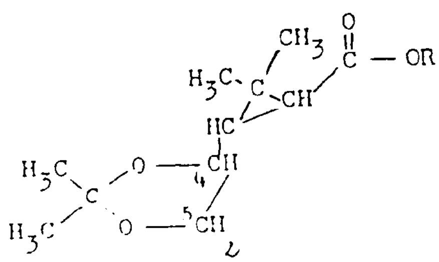

(IIIa)

zu gelangen, worin R wie vorstehend definiert ist und die Konfiguration des Cyclopropanrings die trans-Konfiguration ist, von der man entweder den Dioxolanrest hydrolysiert, um die Verbindung der Formel (IVa)

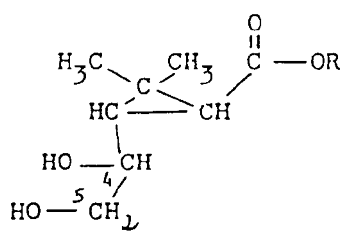

(IVa)

zu erhalten, worin R und die Konfiguration des Cyclopropanrings wie vorstehend definiert sind, und die 4,5-Bindund spaltet, um zu der erwarteten Verbindung der Formel (IA) zu gelangen, oder den Dioxolanrest hydrolysiert und gleichzeitig die 4,5-Bindung spaltet, um die erwartete Verbindung der Formel (IA) zu erhalten.

4. Verfaren gemäß Anpruch 1 zur Herstellung von racemischen oder optisch aktiven Verbindungen der Formel (IB)

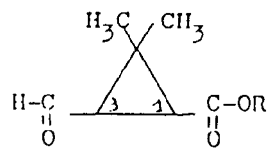

($I_B$)

mit cis-Struktur, worin R wie vorstehend definiert ist, dadurch gekennzeichnet, daß man die Verbindung der Formel (II')

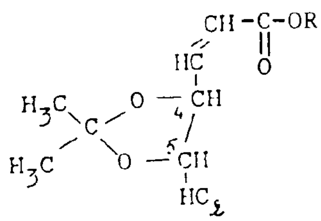

(II')

worin R wie vorstehend definiert ist und die Geometrie der Doppelbindung die Z-Geometrie ist, der Einwirkung von Isopropyliden-diphenylsulfuran unterzieht, um die Verbindung der Formel (IIIb)

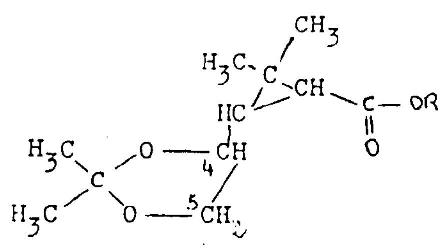

(IIIb)

zu erhalten, worin R wie vorstehend definiert ist und die Konfiguration des Cyclopropanrings die cis-Konfiguration ist, oder den Dioxolanrest hydrolysiert, um die Verbindung der Formel (IVb)

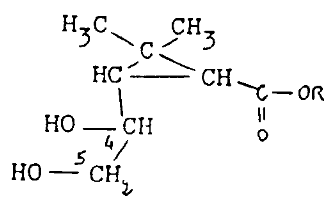

(IVb)

zu erhalten, worin R und die Konfiguration des Cyclopropanrings wie vorstehend definiert sind, und die 4,5-Bindung spaltet, um die erwartete Verbindung der Formel (IB) zu erhalten, oder den Dioxolanrest hydrolysiert und gleichzeitig die 4,5-Bindung spaltet, um die erwartete Verbindung der Formel (IB) zu erhalten.

5. Verfahren gemäß Anspruch 4 zur Herstellung von Verbindungen der Formel (IB) mit (1R,cis)-Konfiguration, dadurch gekennzeichnet, daß man die Verbindung der Formel ($II'_1$)

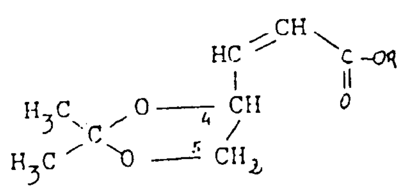

($II'_1$)

mit (4S)-Konfiguration, worin R wie vorstehend definiert ist und die Geometrie der Doppelbindung die Z-Geometrie ist, der Einwirkung von Isopropyliden-diphenylsulfuran unterzieht, um zu der Verbindung der Formel ($IIIb_1$)

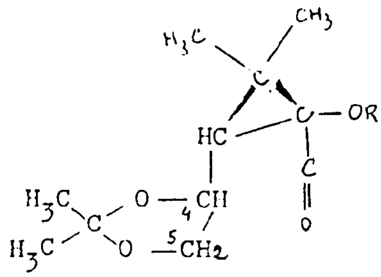

(IIIb$_1$)

mit (4S)- und (1R,cis)-Konfiguration im Hinblick auf den Cyclopropanring zu erhalten, worin R wie vorstehend definiert ist, von der man entweder den Dioxolanrest hydrolysiert, um die Verbindung der Formel (IVb$_1$)

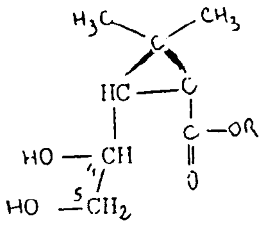

(IVb$_1$)

mit (4S)- und (1R,cis)-Konfiguration im Hinblick auf den Cyclopropanring zu erhalten, worin R wie vorstehend definiert ist, und die 4,5-Bindung durch Einwirkung eines Oxidationsmittel spaltet, um zu der Verbindung der Formel (IB) mit (1R,cis)-Konfiguration zu gelangen, oder den Dioxolanrest hydrolysiert und gleichzeitig die 4,5-Bindung spaltet, um die Verbindung der Formel (IB) mit (1R,cis)-Konfiguration zu erhalten.

6. Verfahren gemäß Anspruch 3 zur Herstellung von Verbindungen der Formel (IA) mit (1R,trans)-Konfiguration, dadurch gekennzeichnet, daß man die Verbindung der Formel (II"$_1$)

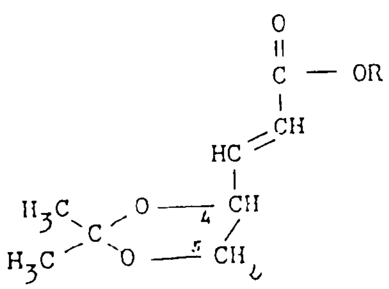

(II"$_1$)

mit (4R)-Konfiguration, worin R wie vorstehend definiert ist und die Geometrie der Doppelbindung die Geometrie ist, der Einwirkung von Isopropyliden-diphenylsulfuran unterzieht, um zu der Verbindung der Formel (IIIa$_1$)

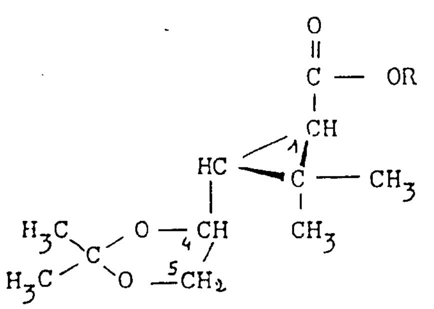

(IIIa$_1$)

mit (4R)- und (1R,trans)-Konfiguration im Hinblick auf den Cyclopropanring zu gelangen, worin R wie vorstehend definiert ist, von der man entweder den Dioxolanrest hydrolysiert, um zu der Verbindung der Formel (IVa$_1$)

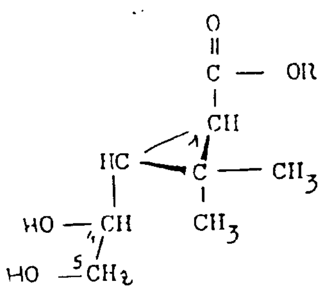

(IVa$_1$)

mit (4R)- und (1R,trans)-Konfiguration im Hinblick auf den Cyclopropanring zu gelangen, worin R wie vorstehend definiert ist, und die 4,5-Bindung durch Einwirkung eines Oxidatiosmittels spaltet, um zu der Verbindung der Formel (IA) mit (1R,trans)-Konfiguration zu gelangen oder den Dioxolanrest hydrolysiert und gleichzeitig die 4,5-Bindung spaltet, um zu der Verbindung der Formel (IA) mit (1R,trans)-Konfiguration zu gelangen.

7. Verfahren gemäß Anpruch 3 zur Herstellung von Verbindungen der Formel (IA) mit (1R,trans)-Konfiguration, dadurch gekennzeichnet, daß man die Verbindung der Formel (II'$_1$)

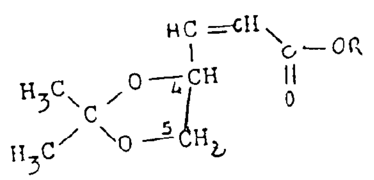

(II'$_1$)

mit (4R)-Konfiguration, worin R wie vorstehend definiert ist und die Geometrie der Doppelbindung die Z-Geometrie ist, der Einwirkung eines Isopropyliden-triphenylphosphorans unterzieht, um zu der Verbindung der Formel (IIIa$_1$), wie vorstehend definiert, zu gelangen, wonach man die Synthese wie in Anspruch 6 fortführt, um das erwartete Produkt der Formel (IA) mit (1R,trans)-Konfiguration zu erhalten.

8. Als neue, industrielle Verbindungen die Verbindungen der Formeln (IIIb) und (IVb), wie in Anspruch 4 definiert.